# EUROPEAN PATENT SPECIFICATION

(11) **EP 0 624 164 B1**
(45) Date of publication and mention of the grant of the patent: **11.02.1998**
(21) Application number: 94901809.7
(22) Date of filing: 16.11.1993
(51) Int. Cl.: A61K 38/10, C07K 14/47

(54) **PEPTIDES WITH ORGANO-PROTECTIVE ACTIVITY, PROCESS FOR THEIR PREPARATION AND THEIR USE IN THE THERAPY**
BPC-PEPTIDE, DEREN HERSTELLUNG UND THERAPEUTISCHEN VERWENDUNG
PEPTIDES A ACTIVITE ORGANO-PROTECTRICE, LEUR PROCEDE DE PREPARATION ET LEUR UTILISATION EN THERAPIE

(30) Priority: 16.11.1992 HR 128392
(43) Date of publication of application: 17.11.1994
(73) Proprietor: Petek, Marijan Mr.Sc., YU-41 000 Zagreb (YU); Seiwerth, Sven, YU-41 000 Zagreb (YU); Sikiric, Predrag Dr.Sc., 41000 Zagreb (YU); Grabarevic, Zeljko, YU-41 000 Zagreb (YU); Rotkvic, Ivo Mr.Sc., YU-41 000 Zagreb (YU); Duvnjak, Marko, YU-41000 Zagreb (YU); Turkovic, Branko, YU-41 000 Zagreb (YU); Mise, Stjepan Mr.Sc., YU-58000 Split (YU); Suchanek, Ernest, Dr.Sc., YU-41000 Zagreb (YU); Mildner, Boris, YU-41 000 Zagreb (YU); UDOVICIC, Ivan, CH-6370 Stans (CH)
(72) Inventor: Petek, Marijan Mr.Sc., YU-41 000 Zagreb (YU); Seiwerth, Sven, YU-41 000 Zagreb (YU); Sikiric, Predrag Dr.Sc., 41000 Zagreb (YU); Grabarevic, Zeljko, YU-41 000 Zagreb (YU); Rotkvic, Ivo Mr.Sc., YU-41 000 Zagreb (YU); Duvnjak, Marko, YU-41000 Zagreb (YU); Turkovic, Branko, YU-41 000 Zagreb (YU); Mise, Stjepan Mr.Sc., YU-58000 Split (YU); Suchanek, Ernest, Dr.Sc., YU-41000 Zagreb (YU); Mildner, Boris, YU-41 000 Zagreb (YU); UDOVICIC, Ivan, CH-6370 Stans (CH)
(74) Representative: Gleiss & Grosse
(86) International application number: EP9303217
(87) International publication number: WO9411394

(56) References cited:
- EP-A- 0 432 400
- EP-A- 0 572 688

## Description

This invention relates to the use of peptides having a high biological activity of the same type as known for natural compound BPC, but with shorter amino acid chains, in the preparation of medicaments for treating various diseases.

### Background of the invention

A biologically active protein with organo-protective activity which was isolated from human or animal body and named BPC (Body protecting Compound) was disclosed in EP 0 432 400 and also published: P. Sikiriç et al., Exp.Clin. Gastroenterol.,1 15-26, 1991. This protein has a high molecular weight of about 40 000 + 5000 Daltons, with only partial determined structure. This compound has a very broad spectrum of biological activities like: ulcer protective, hepatoprotective, anti viral, anti edematous, general anti inflammation activity, anti malignant activity and others. It should be used in the therapy of cited diseases, further in the therapy of diseases and disorders of nerve system, disorders of dopaminergic ethiology, surgery, stomatology, in the curing of infertility and in veterinary medicine. But this broad spectrum of activity can be probably a consequence of undetermined structure or even of insufficient purity or homogeneity of isolated compound BPC.

EP-A-572 688 discloses N-terminal peptides of BPC and particular therapeutic uses of these peptides. This document has not been published prior to the priority date of the present invention and is therefore only relevant for the question of novelty.

### Description of the invention

The present invention, is based on and uses a class of synthetic peptides which exhibit surprisingly high biological activity, especially in the sence of body protection. In any case these synthetical compounds with well defined structure have a great advantage in comparison to the only partially defined high molecular protein BPC, which is obtainable only by a difficult procedure from dubious natural source.

More particularly, this invention is based on and uses a class of biologically highly active peptides, consisting of 8 to 15 amino acid residues, which are represented by the following basic structural formula using the three letter amino acid code and the numbers below each amino acid residue refer to the position of the residue in the peptide chain: 
Xaa Zaa Pro Pro Pro Xaa Yaa Pro Ala Asp Zaa Ala Xaa
1               5                   10
Xaa Xaa
    15
 wherein one or more amino acid residues are substituted. The substiuents Xaa, Yaa and Zaa which may be employed are shown in the following table I.

**Table I**

| Residue | Substituent |
|---|---|
| Xaa | a neutral aliphatic amino acid: Ala,bAla,Leu,Ile,Gly,Val,Nle,Nva |
| Yaa | basic amino acid: Lys,Arg,Orn,His |
| Zaa | acidic amino acid: Glu,Asp,Aad,Apm |

Preferred peptides are as follows:

Seq.Id.No: 1
Leu Glu Pro Pro Pro Gly Lys Pro Ala Asp Asp Ala Leu
1               5                   10
Gly Val
    15

Seq.Id.No: 2
Gly Glu Pro Pro Pro Gly Lys Pro Ala Asp Asp Ala Gly
1               5                   10
Leu Val
    15

Seq.Id.No: 3
Leu Glu Pro Pro Pro Leu Lys Pro Ala Asp Asp Ala Leu
1               5                   10
Gly Val
    15

This invention also uses analogous peptides in amide or carboxy terminated form at C terminus, having the structural formulas herein above described, and wherein at least one and at most 7 amino acid residues in region 1-15 are omitted and at least one of the remaining amino acid residues can be substituted according to the described schema of substitution in table I.

Preferred peptides are as follows:

Seq.Id.No:4

Leu Glu Pro Pro Pro Leu Lys Pro Ala Asp Ala Leu Gly
1               5                   10
Val
14

Seq.Id.No:5

Gly Glu Pro Pro Pro Gly Lys Pro Ala Asp Ala Gly Leu
1               5                   10
Val
14

Seq.Id.No: 6

Glu Pro Pro Pro Leu Lys Pro Ala
1 5 8

Seq.Id.No: 7
Asp Pro Pro Pro Ile Arg Pro Ala Asp
1 5 9

Seq.Id.No: 8

Glu Pro Pro Pro Leu Lys Pro Ala Asp
1 5 9

In accordance with another embodiment, peptides are used having the structural formulas herein above described, and wherein at least one of amino acid residues can be omitted and at least one of the remaining amino acid residues can be substituted as indicated in table I, which are transformed in cyclic form by the formation of a new bond CO-NH between the first and the last amino acid residues in the molecule. Preferred peptides are as follows:

Seq.Id.No: 9

Leu Glu Pro Pro Pro Leu Lys Pro Ala Asp Ala Leu Gly
2 5 10 13

Val

Seq.Id.No: 10

Gly Glu Pro Pro Pro Gly Arg Pro Ala Asp
2 5 9

Applicants have found that when employing the peptides as herein above described, such peptides display a biological activity equal to or greater than the parent protein BPC.

Pharmacological investigation of the described peptides has been done on different common models "in vitro" and "in vivo" and following pharmacological properties were found:

### 1.) Protective effect on liver and pancreas

Protective effect of newly synthesized peptides, coded Seq.Id.No.: 2,3,4,6 have been compared with reference standards (bromocriptin, amantadine, somatostatin) in different experimental models of liver and pancreas injury in the rats: 24h bile duct ligation, 24h bile duct + hepatic artery ligation, 48h restraint stress, CCl₄ application. the used peptide, applied in range between 10 µg - 10 ng/kg b.w., either i.g. or i.p. significantly prevent the liver and pancreas necrosis, as well as fatty changes in animals subjected to 24h bile duct ligation, 24h bile duct + hepatic artery ligation, 48h restraint stress, CCl₄ application. Biochemical values of bilirubin, SGOT, SGPT are completely in line with mentioned macro/microscopically data.

### 2.) The effect on kidney lesions

### a) Unilateral nephrectomy

In experiments Wistar albino rats of both sexes, 190-250 g, were used. A unilateral nephrecromy has been done, and as a result, an increase of the remaining kidney weight has been observed in control as well as in groups treated with peptides Seq.Id.No.2,3,4,6, given in 10 µg/kg b.w.i.p. 1h before surgery. In peptides treated groups a decrease of the remaining kidney raise has consistently been observed.

Biochemical parameters in both groups have been compared. It seems that all of the used peptides ameliorate the function of the remaining kidney.

### b) Gentamycin application

In experiments Wistar albino rats, female were used. Tubular damages were induced with gentamycin application, in the dosage of the 40 mg/kg applied one daily during a period of 30 days. All of the animals were sacrificed 5 days following last drugs application. Significant tubular lesions have been observed in control groups, whereas significantly less microscopically visible lesions were consistently been found in all groups treated with the above mentioned peptides.

### c) Mercury chloride

In experiments Wistar albino rats were used. Mercury chloride was applied in the dosage of the 2mg/kg b.w.i.v., and peptides Seq.Id.No.: 2,3,4,6 in the dose of the 20 µg/kg 1h before mercury chloride application. Unlike control data biochemical as well as microscopical results showed a protective effect in groups treated with mentioned peptides.

### 3.) The influence on testis lesions

The influence of peptides Seq.Id.No.: 2,3,4,6 on testis lesions has been investigated by ketoconasol application (24 mg/kg i.g.), testosterone (30 mg/kg i.p.) or 6 Gy irradiation. Peptides Seq.Id.No.: 2,3,4,6 were given 1h before. Peptides Seq.Id.No.: 2,3,4,6 demonstrated a protective effect.

### 4.) Fertility/Commercial breeding

a) Peptides Seq.Id.No. 2,3,4,6 and fertility: The influence on oligoasthenospermis. The research was performed on 10 men, age 30-40 years, with diagnosis of oligoasthenospermis. From these patients an ejaculate was taken after 3 to 4 days of abstinence. After liquefaction (30min) to the 0.5ml of ejaculate is added in layer 0.5ml of medium. The control medium consisted of HAM-F 10 with 10 % of deactivated chordal serum. Experimental medium contained additionally 2 µg/ml or 4 µg/ml of BPC. After traveling time for 90 minutes at 37°C in atmosphere containing 5% of CO₂, in the Horwell Fertility Chamber was determined the number of progressive movable, movable on place and immovable spermatozoa in 1 ml of ejaculate. Preliminary results show no effect on motility of spermatozoa of lower concentration of the mentioned peptides. However, in their higher concentration (4µg) a significantly % of motility relative to control was determined.

### Peptides Seq.Id.No. 2,3,4,6 and reproduction processes

The mentioned peptides were investigated in mice with previous history of three pregnancies. 20 days after cessation of the last lactation, the animals were subjected to copulation. The used peptides were given in a dose of the 10 µg/kg b.w.i.p. once daily during all period of pregnancy (19-21 days) and lactation (next three weeks). Control group received simultaneously an equivolume of saline. The number of offspring per female and the weight of offspring were recorded.

A careful statistical analysis revealed consistently a significantly increased number of offspring per female, as well as surprisingly, no difference between the body weight at each of the studied time intervals. All of the female were allowed to recover for the next 25 days after cessation of the lactation. Then, they were again subjected to copulation for investigation of the effect on fifth pregnancy.

With regard to control group, an increased fertility rate with increased capability to lactation was registered in all groups treated with the mentioned peptides.

It should be noted that mentioned peptides could improve the fertility in quite old mice. As a conclusion, these results are in line with overall beneficial peptides capacity and in vitro data obtained using human sperm.

### c) Commercial breeding

1419 healthy pigs (from total 4306 pigs) received immediately after birth 10 µg of the peptides Seq.Id.No.2,3,4,6, other 1440 at the 13th day of life, 1h before castration, whereas remaining 1477 received only conventional Fe-therapy. Contusion, culling, mortality, body weight and food consumption were assessed after 4 weeks. The used peptides, applied once significantly decrease culling rate (in the groups treated with peptides immediately after the birth) and contusion rate (in all groups treated with peptides). The same body weight was obtained in peptides treated animals with markedly lower consumption of food. Influence on infection: 40 weaned pigs affected with E.coli enterocolitis, 28 days old, were investigated. The used peptides were applied in 20 animals (10 µg/kg b.w.i.m.). One month later a significantly lowering of mortality rate was noted in peptide-treated animals.

In another experiment mortality rate after the first week was investigated in a total number of 1200 healthy pigs. 400 animals received the mentioned peptides immediately after the birth, in addition to conventional fe-thereapy. A significantly lower mortality was noted in mentioned peptide-treated (10 µg/kg b.w.i.m.) groups.

### 5.) Influence on amphetamine behavior

The influence of peptides Seq.Id.No.: 2,3,4,6 was investigated in a dosage of the 10 µg/kg or 10 ng/kg b.w.i.p., 5 minutes before or simultaneously with amphetamine application. In the groups treated with the mentioned peptides a significant decrease of the amphetamine induced behavioral disturbances was noted.

### 6.) Bleeding time

The influence of the peptides Seq.Id.No. 2,3,4,6 was investigated by usual bleeding assays (tail cutting or incision) in mice and rats, with or without heparin application (1000 UI/kg s.c. 3h before bleeding). The investigated peptides (10 µg/kg or 10 ng/kg i.p.) were applied simultaneously with bleeding induction. In all animals treated with studied peptides a significant decrease of the bleeding time was noted.

### 7.) The influence on consequences of castration

A modified procedure according to Allen-Doisy method has been done. Different dosages were applied (10 µg or 10 ng/kg i.p.), in single application or in once daily regimen, as pre treatment or post treatment. In groups treated with the investigated peptides a prevention or reversion of different castration consequences (e.g., vaginal epithelium atrophy, oesteoporosis).

### 8.) Hypertension

Hypertension was induced by unilateral renal artery occlusion. After 10 days, the animals were treated with peptides Seq.Id.No. 2,3,4,6 (10 µg or 10 ng/kg b.w.i.p.) or saline, using various protocols (single or continuos application). In groups treated with the studied peptides a significant decrease of the raised blood pressure values was observed.

### 9.) The influence on experimental diabetes mellitus

Wistar male albino rats, 175-230 g b.w., were used for streptozotocin or alloxan induced diabetes mellitus. Peptides Seq.Id.No. 2,3,4,6, applied in the dosage of the 10 or 100 µg/kg significantly delay the diabetes onset in streptozotocin treated animals. Moreover, they significantly prolonged the survival time.

### 10.) The influence on thyreoparathyreoidoctomy

A total ablation of thyreoparahtyorids glands in Wistar rats has been performed. Thereafter, peptides Seq.Id.No. 2,3,4,6 (10 ng/kg b.w.i.p.) or saline (5 ml/kg b.w.i.p.) using different protocols (single or repeated treatment). In groups treated with the studied peptides the consequences of glands removal have significantly been reduced.

### 11.) Carotid artery legation

Animals were sacrificed 3 hours after both carotid arteries legation. One hour before all of the animals were treated with peptides Seq.Id.No. 2,3,4,6 (10 n/kg b.w.i.p.) or saline (5 ml/kg b.w.i.p.). In investigated peptide-treated groups significantly less brain edema has been found.

### 12.) Adrenal glands

For adrenal glands injury induction the animals received aniline (300 mg/kg s.c. during 7 days). All of the animals were simultaneously treated with peptides Seq.Id.No.: 2,3,4,6 (10 µg/kg b.w., 10 ng/kg b.w.i.p.) or with saline (5 ml/kg b.w.i.p.). The weight of the adrenals and microscopically changes were assessed. Relative to aniline control group, the investigated peptides significantly reduced the aniline-induced increase of the adrenal glands weight in both dosages. Microscopically visible protection was dose dependent. As it is in generally accepted, that aniline induced disturbances are related to ACTH hyper secretion, a causal relationship could be established between studied peptides and ACTH. This seems to be also supported by the finding of the same weight in groups treated with the mentioned peptides as in the native healthy control group.

Additionally, the animals were subjected to 48 h restraint stress procedure, after the pretreatment with investigated peptides (10 µg or 10 ng/kg i.p./i.g.). A decrease of the raised weight of the adrenals in the studied peptides treated animals was consistent with corresponding microscopically observation.

### 13.) Temperature disturbances

An increase of body temperature was provoked by application of yeast solution (4000 mg/kg s.c.), and a temperature decrease was induced using an immersion in cold water, temperature 4oC, or reserpine application (5 mg/kg i.p.). Different regimens of peptides Seq.Id.No. 2,3,4,6 (10 ng/kg b.w.i.p.) investigated on both increased as well as decreased temperature, have consistently been demonstrated to normalize both temperature disturbances.

### 14.) Influence on hematopoeasis

Albino mice were injected with cytostatics (Endoxan, Vincristin, Adriablastin, Cytosin-arabinosid) in LD₅₀ doses. One hour before cytostatic application, the animals were treated with peptides Seq.Id.No. 2,3,4,6 in a dose of 10 µg, whereas controls received an equivolume of the saline. The animals were sacrificed on 3,5,7 and 11th day of experiment. Parameters in blood (E, Hb, Htc, L, Tb abs. number of neutrophiles), bone marrow, cytology and histology of liver and spleen were assessed.

On the third day of experiment no difference between the groups were observed in L,E,Tb and neutrophiles values, unlike intact haematopoetic cells in bone marrow of the animals treated with peptides, strongly contrasting with aplasia noted in the controls.

The number of neutrophiles and leukocytes significantly increased in animals treated with peptides till fifth day, reaching the normal values till seventh day, unlike controls where normalization should not be observed before 11th day.

### 15.) Pain reduction

Pain was induced by an intraperitoneal application (0.2 ml/mouse) 2% MgSO4 or 0.6% acetic acid. Various regimens of peptides Seq.Id.No. 2,3,4,6 relative to the dose or time of application (10 µg or 10 ng/kg i.p., simultaneously or 30 min before) significantly reduce the writhing time in comparison with the control groups.

### 16.) Infection induced by Rickettsias

0.2 ml of suspension of Coxielle Burnetti, dissolved 10-9, was injected in the suitable eggs. In the eggs, the peptides Seq.Id.No. 2,3,4,6, (1 pg, 10 pg, 100 pg and 1 ng) were also injected. The survival time was significantly longer in the treated groups than in the controls.

### 17.) Influence on tumor cells

a) Two cell lines L-924 and melanoma B-16 were cultivated in vitro under standard conditions. In vitro the influence of peptides Seq.Id.No. 2,3,4,6 on growing of the mentioned cell cultures was investigated. Two days following the initiation of cell cultures, mentioned peptides were added in 3 % concentration (0.1 ml). Three days later the numbers of cells per cultures were determined by cell counter. The results showed an inhibitory effect of the mentioned peptides on B-16 melanoma cells.
b) Erlich's ascites tumor (EAT) is the tumor which can grow in all strains of mice, either as ascitic or solid, depending upon way of administration of tumor cells. The influence of incubation of EAT cells with peptides Seq.Id.No. 2,3,4,6 on survival time of mice, injected with these cells subcutaneously or intraperitoneally, was investigated. The animals were observed during the period of 45 days. Control of 15 male mice, NMRI strain, received 0.4x106 EAT cells. Prior to the injection tumor cells were incubated for one hour at 4°C in saline. Volume of injection was 0.2 ml. Median survival time in this group was 36 days, and only 3/15 survived 45 days.
   Experimental group of 15 NMRI male mice received the same dose of tumor cells, in the same volume, but these cells were previously incubated in mentioned peptides solution (2 µg/ml). Only 2/15 mice died during the observed period (45 days), where all others survived more than 45 days. Difference between the control and the experimental group was significantly different (P<0.01). The same results were observed if the same procedure was carried out and the EAT injected i.p. (instead s.c.).
c) The mice were used for investigation of the influence of peptides Seq.Id.No. 2,3,4,6 (given in a dose of the 10 µg/kg b.w.i.p.) on lung metastases induced by melanoma or aplastic mamas cercinoma cells injection. In peptide-treated groups a significant decrease of lung metastases number was determined.

Based on the above described data, it could be stated that the mentioned peptides prolonged the survival time of the animals with tumors, showing an anti tumor activity.

### 18.) Anti depression activity

For revealing of antidepressant activity the forced swimming test according Porsolt et al, Eur. J. Pharmacol., 47, 379-391, 1978, was used.

Male Wistar rats (180-240 g b.w. have been used for this experiment. The investigated peptide Seq.Id.No:4 has been applied according to the described protocol. Briefly, control animals received saline, the assessment were for 5 min, and the time of immobility has been calculated. The immobility time in the control was 150 sec, whereas in the peptide treated animals the values of only 60-70 sec have been observed. A dose response curve could be calculated as follows: 10 µg - 10 ng/kg full effect, 10 pg/kg b.w. the activity still present, 1 pg/kg the effect not present.

The following results of pharmacological investigation of these peptides exhibit activity in the sense to protect organism against stress and diseases, in general to normalize the organic functions.

Their use should be also effective for prevention and therapy of serveral human or animal diseases and disorders. More particularly, these compounds will be used for the treatment of:
- disturbances and lesions of liver and pancreas
- atrophy of testis and sperms mobility
- fertility disturbances
- commercial breeding improvement
- rickettsias illnesses
- thyreoparathyreoidoctomy
- diabetes mellitus
- disturbances of adrenal gland
- disturbances of hematopoetic system
- disturbances of coagulation
- disturbances of bleeding
- post-castration/menopausal disturbances
- ischemic and toxic lesions
- psychiatric disturbances
- hypertension
- disturbances of body temperature
- pain
- tumor

In general, the described peptides can also be employed in a wide variety of pharmaceutical compositions in combination with a non-toxic pharmaceutical carrier or vehicle such as a filler, non-toxic buffer, or physiological saline solution. Such pharmaceutical compositions can be used topically or systematically and can be in any suitable form such as a liquid, solid, semisolid, injectable solution, tablet, ointment, lotion, capsule, lingualette or similar.

These peptides will be in general administered in a dosage of from 10⁻⁵ to 10⁻² mg/kg of body weight, when applied systematically. When administered topically, they will be used in higher concentration, e.g. 0.1% to 0.5%.

Very favorable is the absence of any signs of toxicity till doses of 50 mg/kg b.w. and also good activity of compounds by peroral administration (intra gastric).

The peptides described herein can be synthesized using step by step condensation of protected amino acides in homogenous liquid system or preferable using solid phase method. For preparation of cyclic peptides partially protected linear peptides of desired length are prepared as with alkyl ester group on C-terminus, which is converted into azide, following coupling and deprotecting. Alternatively, partially protected linear peptide with free terminal groups can be cycled by diphenylphosphorylazide in very diluted solution.

### Example 1

### Peptide synthesis using Boc strategy:

Peptide synthesis was performed beginning with 100 mg of Boc-Val-PAM resin to produce C-terminal carboxyl peptide (PAM purchased from Applied Biosystems, substitution 0.56 meq/g is an amino-acyl-4-(oxymethyl)-phenylacetic acid derivative of aminopolystyrene). Boc amino acids (Boc = tert.-butoxycarbonyl-) were condensed one after other on polymer carrier using diisopropylcarbodiimide (DIPC) as condensation reagent. In each step Boc group was removed with 50 % solution of trifluoroacetic acid (TFA) in dichloromethane. Amino group was then deprotonated with diisopropylethylamine.

The conversion in each step should be higher than 99.5%. If not, the condensation was repeated. After complete synthesis, cleavage was performed using low HF procedure (2 hours at 0°C). As carbonium ion scavenger was used anisole. HF was evaporated by a flow of nitrogen. Crude raw peptide was then obtained by pouring of oily residue into dry ether.

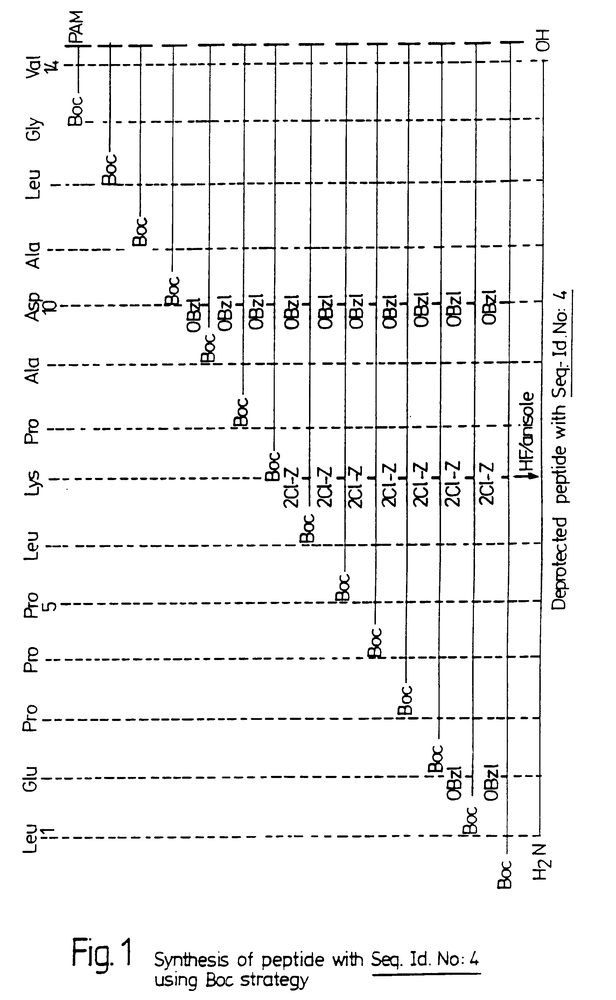

Then the raw peptide was purified by reverse phase HPLC using column 5 x 150 mm filled with silica gel RP-18, gradient elution with solvent system: 0.1% TFA in water/acetonitrile. Detection: ultraviolet absorption at 225 nm. The synthesis of the peptide with Seq.Id.No: 4 is depicted in Fig. 1.

### Example 2.

### Peptide synthesis using Fmoc strategy:

Standard Fmoc protected amino acids were used for the synthesis (Fmoc = 9-fluorenylmethyloxy-carbonyl-). The side chain fuctions were protected as 0-tetra. butyl esters (Asp, Apm, Glu, Aad) and as Boc derivatives (Lys). First amino acid (Val) was bonded on to polymeric carrier-BHA resin (BHA = benzhydrilamino resin) using diisopropylcarbodiimide as coupling reagent. In each step the Fmoc protective group was removed with piperidine. Thereafter the second and so on all next amino acids were introduced using the same way until the synthesis is completed. The cleavage has been done by a mixture of TFA/TFMSA/anisole = 2:17:52 (vol/vol).

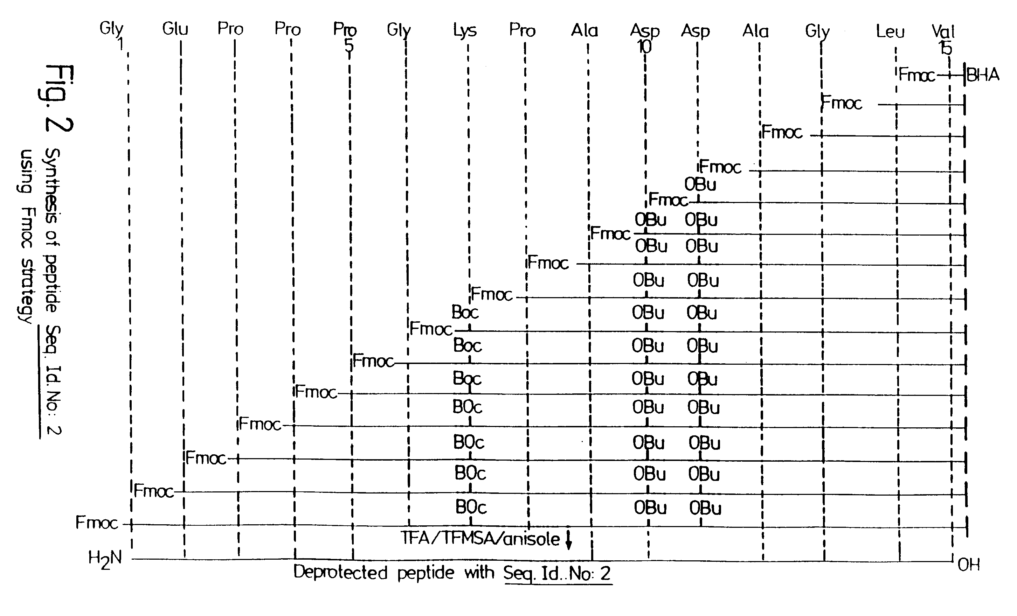

Peptide was then purified using the HPLC method described in example 1. The synthesis of the peptide with Seq.Id.No: 2 is illustrated on the Fig. 2.

### Example 3.

### Peptide synthesis using Ddz strategy:

All amino acids were used protected at their alpha-amino function by Ddz (= alpha, alpha-dimethyl-3, 5 dimethoxybenzyloxycarbonyl-). The side functions were protected by Z group (= benzyloxycarbonyl-) for lysine and 0-t-Bu group (t-butylester) for aspartic and glutamic acid.

A Merrifield support (cross linked chloromethylated polystyrene gel) with capacity of 1.4 mmol/g was used for bonding of the first Ddz-amino acid via its cesium salt. After condensation with dicyclohexylcarbodiimide (DCC) was removed the Ddz protection group in each step with 5% TFA in dichloromethane, following by washing and deprotonation with 10 % triethylamine in dicloromethane. After deprotonation the next coupling step was mediated, using the same method. These coupling steps were repeated until peptide sequence was completed.

Finally the peptide was cleaved from polymeric carrier using a mixture HBr/TFA/anisole. After evaporation of volatile part the just cleaved peptide was precipitated from dry ether and dried. The raw peptide was then purified by HPLC method as described in example 1.

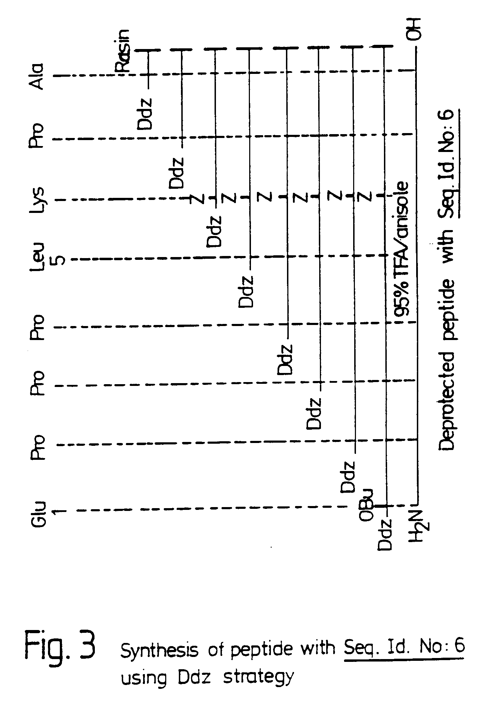

The synthesis of the peptide with Seq.Id.No.: 6 is illustrated on the Fig. 3.

### Example 4.

### Synthesis of cyclic peptide:

Peptide in partially protected form: 
OBzl                    NO2          OBzl

Gly-Glu-Pro-Pro-Pro-Gly-Arg-Pro-Ala-Asp-OH
 was previously prepared according to the method described in examples 1 or 2.

The 0.0005 molar solution of this peptide in dimethylformamide was cycled after addition of diphenylphosphorylazide and triethylamine at 20°C for 12 hours.

Then this mixture was hydrogenated with hydrogen/palladium charcoal catalyst at 25°C for 8 hours.

The solvent was carefully evaporated and crude product purified using HPLC method described in example 1. 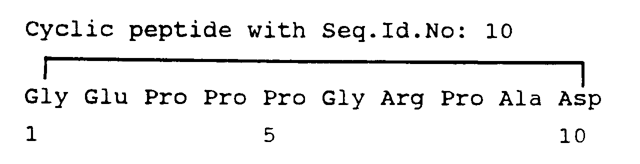
Cyclic peptide with Seq.Id.No: 10
Gly Glu Pro Pro Pro Gly Arg Pro Ala Asp
1               5                   10
 was obtained in 10% yield.

### Synthesis of linear and cyclic peptides using the Ddz strategy:

The same strategy with the Ddz protecting groups was used for obtaining of linear and cyclic peptides. The side functions were protected with Z group (lysine) and with O-benzyl ester groups - OBzl - (glutamic and aspartic acid). Polymeric carrier was HYCRAM^{R} resin (trademark of ORPEGEN, Heidelberg, Germany), which is 4-bromocrotonyl-beta-alanylamidomethyl-polystyrene. The first amino acid (Ddz-valine) was bonded onto polymer carrier via its cesium salt.

Then the Ddz group was removed using trifluoroacetic acid (5%) in dicloromethane and amino group deprotonated with diisopropylethylamine. In the next step Ddz-glycine was coupled onto valine residue in polymer matrix using diisopropylcarbodiimide (DIPC) in the presence of 1-hydroxybensotriazole.

These steps were repeated until peptide chain was completed. The synthesized peptide in protected form was then cleaved carefully from HYCRAM^{R} carrier with tetrakis (triphenyl-phosphino)-palladium (O) dissolved in anhydrous tetrahydrofuran in absence of oxygen. An additive like morpholine was used as acceptor molecule for allylic groups.

Polymeric carrier was filtered off and washed with tetrahydrofurane. The solution of peptide was then filtered through a short column of silica gel to remove the palladium catalyst.

The eluate which contains the partially protected peptide 4a. was dried after evaporation of solvent in vacuum.

### Synthesis of linear peptide with Seq.4

Partially protected peptide 4a. was dissolved in 2,2,2 trifluoroethanol and hydrogenated in the presence of palladium on charcoal (10%) with hydrogen gas at 30°C. The catalyst was filtered off and solvent evaporated in vacuum. The crude residue was then purified using HPLC method as described in example 1. After purification the peptide with Seq.Id.No: 4 was obtained. The product was identical with compound obtained in example 1.

### Synthesis of cyclic peptide with Seq.Id.No: 9

Partially protected peptide 4a was dissolved in dimethylformamide/dichloromethane (1:1) to form 0.001 molar solution. For cyclisation DIPC and HOBt were added and left to stand at 20°C for 10 hours. Then the solution was concentrated to small volume, diluted with 2,2,2trifluoroethanol and purified by passing of column filled with Sephadex LH-20. Fractions containing the partially protected cyclic peptide 9a were collected and hydrogenated with palladium on charcoal (10%) catalyst at 30°C by hydrogen bubbling and intensive shaking for 8 hours.

Then the catalyst was removed by filtration and solution dried by solvent evaporation in vacuum. The crude raw peptide was additionally purified by HPLC using the described method from example 1. The obtained pure cyclic peptide was identified as peptide with Seq.Id.No: 9.

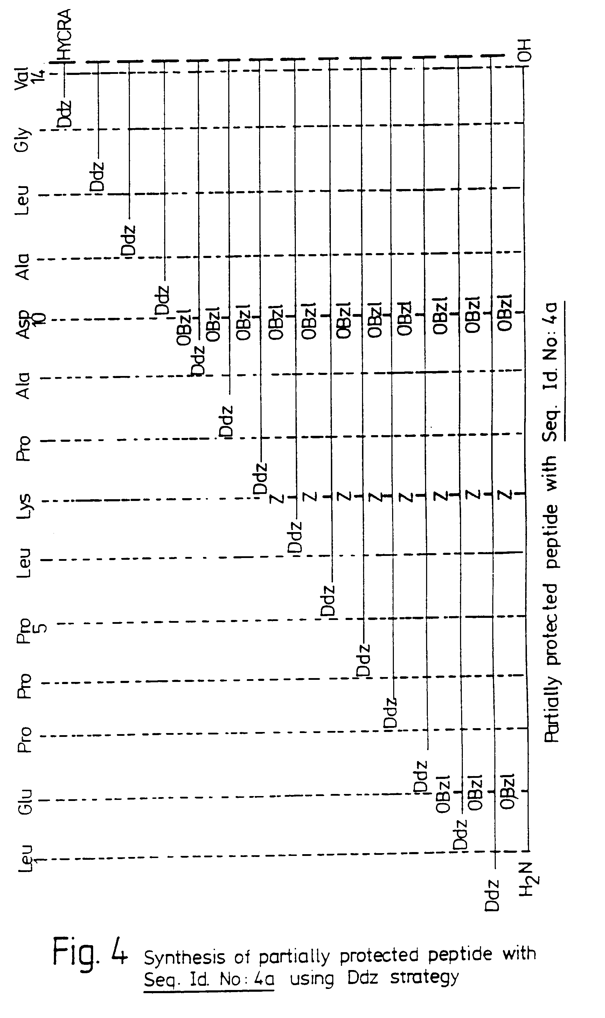

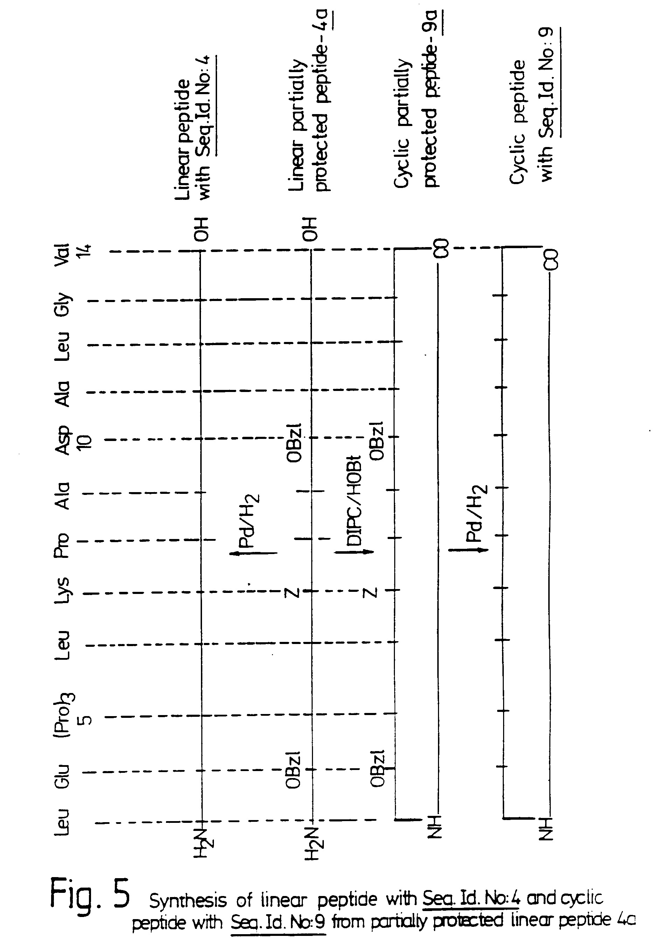

The synthesis on HYCRAM^{R} polymer carrier using Ddz strategy and cyclisation were illustrated on Fig. 4 and 5. All synthesized peptides were checked on their purity by HPLC method using column with silica gel RP-18 (octadecylsilanized), eluted with a mixture of solvents consisting of water/acetonitrile/trifluoroacetic aced gradiently in usual mode. In all cases the purity was higher than 95%.

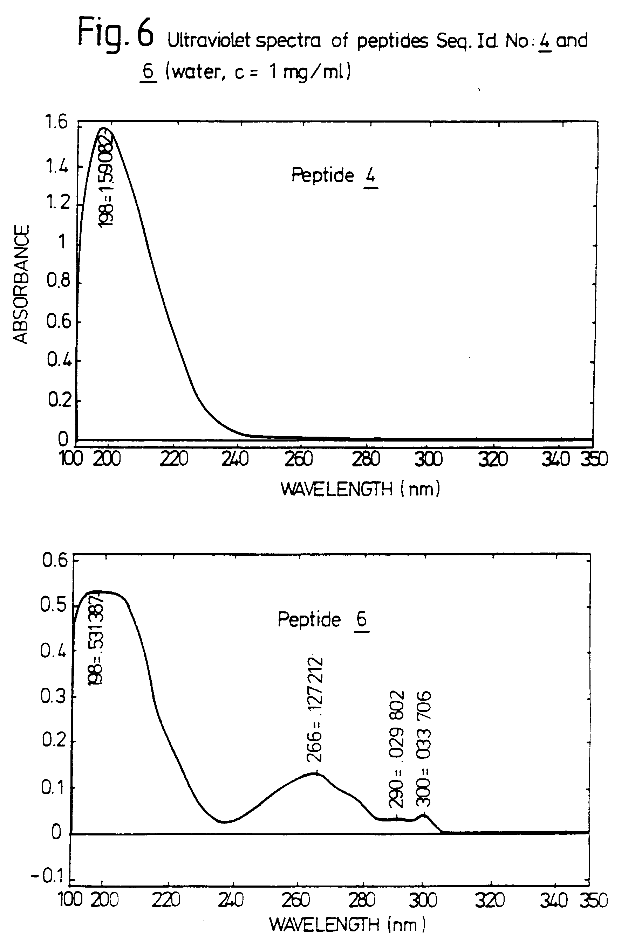

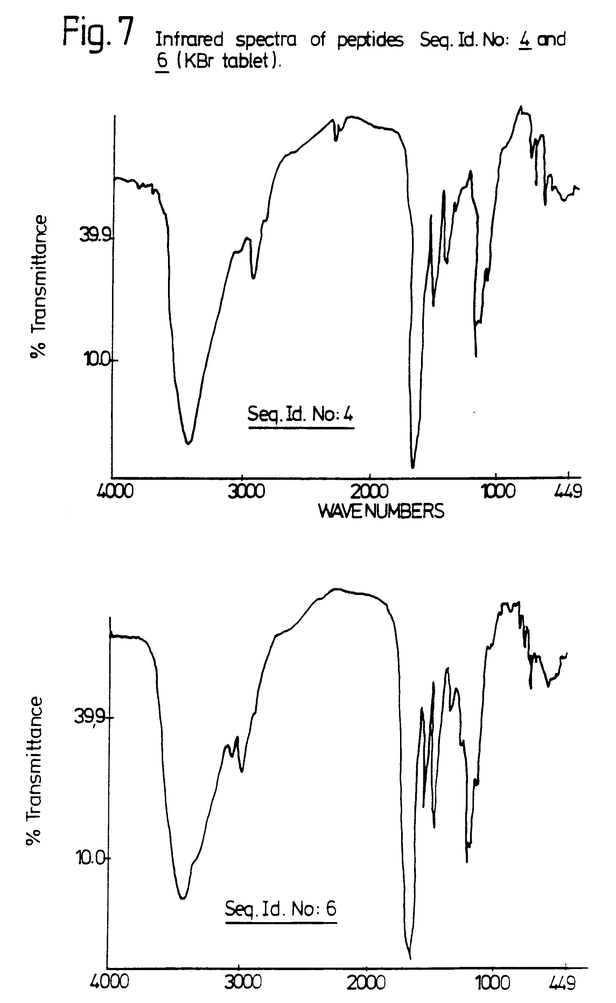

Peptides were characterized with amino acid analysis (values were within +-10% of theory), sequence analysis, molecular weight determined by mass - FAB spectrometry, ultraviolet and infrared spectra (Fig.6 and 7).

## Claims

1. The use of biologically highly active peptides consisting of 8 to 15 amino acid residues and being represented by the general formula: 
Xaa Zaa Pro Pro Pro Xaa Yaa Pro Ala Asp Zaa Ala Xaa
1               5                   10
Xaa Xaa
    15
 in which:
Xaa signifies a neutral aliphatic amino acid residue like:
Ala, bAla, Leu, Ile, Gly, Val, Nle, Nva
Yaa signifies a basic amino acid residue like:
Lys, Arg, Orn, His
Zaa signifies an acidic amino acid residue like:
Glu, Asp, Aad or Apm;
wherein at least one of residues Xaa or Zaa can be omitted, and/or any one of peptides with Seq.Id.No: 1 to 11 for the preparation of a pharmaceutical composition for treating disturbances and lesions of liver kidney and pancreas, atrophy of testis and sperms mobility, fertility disturbances, commercial breeding improvement, rickettsias illnesses, thyreoparathyreoidoctomy, diabetes mellitus, disturbances of adrenal gland, disturbances of hematopoetic system, disturbances of coagulation, disturbances of bleeding, post-castration/menopausal disturbances, ischemic and toxic lesions, psychiatric disturbances, hypertension, disturbances of body temperature, pain or tumor.

2. The use according to claim 1, wherein the peptide is cyclized by means of an amide linkage between the first and last amino acid residue.

## Patentansprüche

1. Verwendung biologisch hochaktiver Peptide umfassend 8 bis 15 Aminosäurereste mit der allgemeinen Formel: 
Xaa Zaa Pro Pro Pro Xaa Yaa Pro Ala Asp Zaa Ala Xaa
1               5                   10
Xaa Xaa
    15
 in der:
Xaa einen neutralen, aliphatischen Aminosäurereste wie:
Ala, bAla, Leu, Ile, Gly, Val, Nle, Nva
Yaa einen basischen Aminosäurerest wie:
Lys, Arg, Orn, His
Zaa einen sauren Aminosäurerest wie:
Glu, Asp, Aad oder Apm bedeutet;
und in der wenigstens einer der Reste Xaa oder Zaa fehlen kann, und/oder eines der Peptide mit den Seq.Id.Nr.1 bis 11 zur Herstellung einer pharmazeutischen Zusammensetzung zur Behandlung von Störungen und Schädigungen der Leber, Nieren und Pankreas, Atrophie der Hoden und Bewegungsfähigkeit des Sperma, Fruchtbarkeitsstörungen, Verbesserung in der kommerziellen Züchtung, Rickettsie, Schilddrüsen/Nebenschilddrüsenentfernung, Diabetes mellitus, Störungen der Nebenniere, Störungen des hämopoetischen Systems, Gerinnungsstörungen, Blutungsstörungen, Störungen nach der Kastrierung und der Menopause, ischämische und toxische Schädigungen, psychiatrische Störungen, Bluthochdruck, Körpertemperaturstörungen, Schmerzen oder Tumor.

2. Verwendung nach Anspruch 1, wobei das Peptid mittels einer Amidbindung zwischen dem ersten und dem letzten Aminosäurerest zyklisiert ist.

## Revendications

1. Utilisation de peptides très actifs biologiquement constitués de 8 à 15 résidus d'acide aminé et qui sont représentés par la formule générale : 
Xaa Zaa Pro Pro Pro Xaa Yaa Pro Ala Asp Zaa Ala Xaa Xaa Xaa
1               5                   10                  15
 dans laquelle :
Xaa indique un résidu d'acide aminé aliphatique neutre tel que :
Ala, bAla, Leu, Ile, Gly, Val, Nle, Nva
Yaa indique un résidu d'acide aminé basique tel que :
Lys, Arg, Orn, His
Zaa indique un résidu d'acide aminé acide tel que :
Glu, Asp, Aad ou Apm ;
où au moins un des résidus Xaa ou Zaa peut être supprimé, et/ou de l'un quelconque des peptides ayant la Seq. Id. n° 1 à 11, pour la préparation d'une composition pharmaceutique pour traiter des troubles et des lésions du foie, du rein et du pancréas, l'atrophie des testicules et la mobilité du sperme, des troubles de la fécondité, l'amélioration de l'élevage commercial, les maladies dues à la rickettsie, la thyréoparathyroïdectomie, les diabètes sucrés, les troubles de la glande surrénale, les troubles du système hématopoïétique, les troubles de la coagulation, les troubles d'hémorragie, les troubles de la post-castration/ménopause, les lésions ischémiques et toxiques, les troubles psychiatriques, l'hypertension, les troubles de la température du corps, la douleur ou une tumeur.

2. Utilisation selon la revendication 1, dans laquelle le peptide est rendu cyclique à l'aide d'une liaison amide entre le premier et le dernier résidu d'acide aminé.
